(19) Europäisches Patentamt
European Patent Office
Office européen des brevets

(11) Veröffentlichungsnummer: **0 048 375**
**B1**

(12) # EUROPÄISCHE PATENTSCHRIFT

(45) Veröffentlichungstag der Patentschrift: **07.09.83**

(21) Anmeldenummer: **81107016.8**

(22) Anmeldetag: **07.09.81**

(51) Int. Cl.³: **C 07 C 63/70**, C 07 C 51/00 // C07C45/46, C07C45/63, C07C51/29

(54) **Verfahren zur Herstellung von 3-Brom-4-fluor-benzoesäure.**

(30) Priorität: **19.09.80 DE 3035355**

(43) Veröffentlichungstag der Anmeldung: **31.03.82 Patentblatt 82/13**

(45) Bekanntmachung des Hinweises auf die Patenterteilung: **07.09.83 Patentblatt 83/36**

(84) Benannte Vertragsstaaten: **AT BE CH DE FR GB IT LI NL**

(56) Entgegenhaltungen: **GB-A-1 088 788** **US-A-2 394 268**

(73) Patentinhaber: **BAYER AG, Zentralbereich Patente, Marken und Lizenzen, D-5090 Leverkusen 1, Bayerwerk (DE)**

(72) Erfinder: **Maurer, Fritz, Dr., Roeberstrasse 8, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 048 375 B1

BUNDESDRUCKEREI BERLIN

Verfahren zur Herstellung von 3-Brom-4-fluor-benzoesäure

Die Erfindung betrifft ein neues Verfahren zur Herstellung von 3-Brom-4-fluor-benzoesäure.

Es ist bereits bekanntgeworden, daß man 3-Brom-4-fluor-benzoesäure erhält, wenn man 3-Brom-4-amino-toluol in Gegenwart von Tetrafluoroborsäure diazotiert, das hierbei gebildete 3-Brom-toluol-4-diazonium-tetrafluoroborat pyrolysiert und das so erhaltene 3-Brom-4-fluor-toluol mit Kaliumpermanganat oxidiert (vgl. Can. J. Chem. 38 [1960], 2441–2449). Dieser in der Literatur beschriebene Syntheseweg ist jedoch für technische Zwecke wenig geeignet, da er eine Reihe von komplizierten Syntheseschritten unter Verwendung von teuren Reaktionskomponenten beinhaltet und nur geringe Ausbeuten erzielt werden können.

Es wurde nun ein Verfahren zur Herstellung von 3-Brom-4-fluor-benzoesäure der Formel I

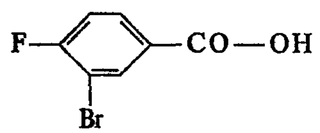

(I)

gefunden, das dadurch gekennzeichnet ist, daß man Fluorbenzol der Formel II

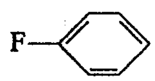

(II)

mit Acetylchlorid in Gegenwart eines Acylierungskatalysators bei Temperaturen zwischen 0 und 100° C umsetzt, das hierbei erhaltene Reaktionsgemisch mit Brom bei Temperaturen zwischen 50 und 150° C umsetzt und das Bromierungsprodukt nach vorheriger Isolierung mit Chlorlauge bei Temperaturen zwischen 0 und 100° C umsetzt.

Überraschenderweise kann 3-Brom-4-fluor-benzoesäure nach dem erfindungsgemäßen Verfahren unter Verwendung billiger Reaktionskomponenten auf einfache Weise in hoher Ausbeute erhalten werden.

Die beim erfindungsgemäßen Verfahren ablaufenden Reaktionen können durch folgendes Formelschema skizziert werden:

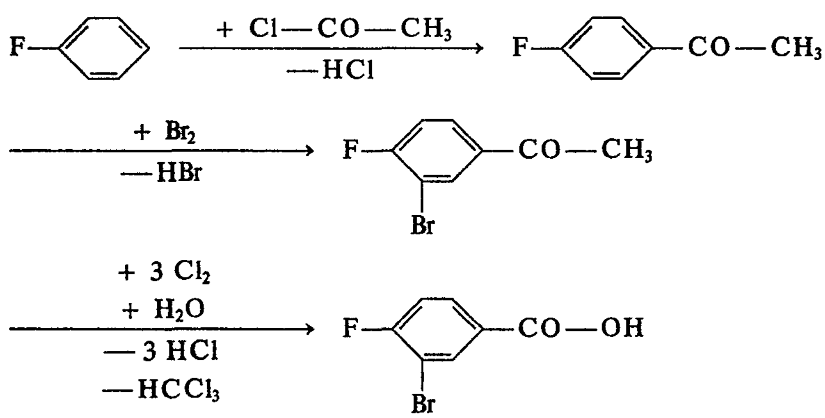


Die erste Stufe des erfindungsgemäßen Verfahrens wird unter Verwendung der üblichen literaturbekannten Acylierungskatalysatoren durchgeführt (vgl. Methodicum Chimicum Band 5 [1975], S. 342–343, Georg-Thieme-Verlag Stuttgart/Academic Press New York-San Francisco-London).

Vorzugsweise wird Aluminiumchlorid als Katalysator eingesetzt.

Je Mol Fluorbenzol werden 0,5 bis 2 Mol, vorzugsweise 1,0 bis 1,6 Mol Acylierungskatalysator und 0,9 bis 1,5 Mol, vorzugsweise 1,0 bis 1,2 Mol Acetylchlorid, im weiteren Verlauf zwischen 0,9 und 2 Mol, vorzugsweise zwischen 1,1 und 1,8 Mol, Brom eingesetzt. Die Chlorlauge wird in der letzten Stufe in einer Menge eingesetzt, die zwischen 2 und 6 Mol, vorzugsweise zwischen 3 und 5 Mol aktives Chlor enthält.

Die Reaktionstemperatur wird in der Acylierungsstufe zwischen 0 und 100° C, vorzugsweise zwischen 20 und 80° C, in der Bromierungsstufe zwischen 50 und 150° C, vorzugsweise zwischen 80 und 120° C, und in der abschließenden Haloform-Reaktion zwischen 0 und 100° C, vorzugsweise zwischen 20 und 80° C gehalten.

Alle Stufen des erfindungsgemäßen Verfahrens werden bei Normaldruck oder geringfügig erhöhtem oder vermindertem Druck, d. h. im allgemeinen zwischen 0,1 und 10 bar, vorzugsweise

zwischen 0,5 und 5 bar durchgeführt.

In einer bevorzugten Ausführungsform des erfindungsgemäßen Verfahrens werden das Fluorbenzol und der Acylierungskatalysator vorgelegt und das Acetylchlorid wird langsam dazu gegeben. Wenn die Umsetzung dieser Komponenten abgelaufen ist, wird bei leicht erhöhter Temperatur Brom zudosiert und die Mischung nach kurzem Nachrühren auf Eis gegeben. Das Bromierungsprodukt, welches man hierbei in kristalliner Form erhält, wird durch Absaugen abgetrennt und noch feucht zu technischer Chlorlauge gegeben. Dieses Reaktionsgemisch wird bei leicht erhöhter Temperatur bis zum Ende der Umsetzung gerührt.

Die Aufarbeitung kann beispielsweise wie folgt durchgeführt werden: Man trennt die wäßrige Lösung vom entstandenen Chloroform ab, versetzt sie mit Natriumbisulfitlösung und stellt durch Zugabe von Salzsäure den pH-Wert auf 2 ein. Das hierbei kristallin anfallende Proukt wird durch Absaugen isoliert, getrocknet und durch seinen Schmelzpunkt charakterisiert.

Die nach dem erfindungsgemäßen Verfahren erhältliche 3-Brom-4-fluor-benzoesäure kann zur Herstellung des als Zwischenprodukt für Insektizide bekannten 3-Phenoxy-4-fluor-benzylalkohols (vgl. DE-OS 2 709 264) verwendet werden.

Hierzu wird zunächst aus 3-Brom-4-fluor-benzoesäure (bzw. deren Natrium- oder Kaliumsalz) durch Umsetzung mit Kaliumphenolat in Gegenwart eines Kupferkatalysators, wie z. B. Kupfer(II)oxid, und unter Verwendung von Phenol als Verdünnungsmittel bei Temperaturen zwischen 150 und 200°C 3-Phenoxy-4-fluor-benzoesäure hergestellt. Hieraus erhält man 3-Phenoxy-4-fluor-benzylalkohol durch Umsetzung mit einem starken Reduktionsmittel, wie z. B. Lithiumalanat, bei Temperaturen zwischen 0 und 100°C, gegebenenfalls unter Verwendung eines Verdünnungsmittels, wie z. B. Tetrahydrofuran (vgl. DE-OS 2 915 738/Le A 19 590).

## Beispiel

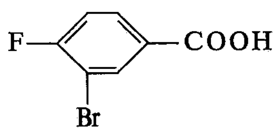

Zu einer Mischung aus 96 g (1 Mol) Fluorbenzol und 200 g (1,5 Mol) Aluminiumchlorid tropft man bei 30—35°C unter schwachem Kühlen 82,5 g (1,05 Mol) Acetylchlorid. Man läßt das Gemisch 1 Stunde bei 50—60°C ausreagieren und gibt dann bei 90—100°C 184 g (1,5 Mol) Brom zu. Anschließend rührt man 1 Stunde bei 90—100°C nach und gibt dann die noch heiße Mischung auf Eis. Das ausgefallene Produkt wird abgesaugt und mit Wasser nachgewaschen und dann noch feucht zu 1,7 l technischer Chlorlauge gegeben. Man rührt das Gemisch zunächst 1 Stunde bei Raumtemperatur nach und erwärmt es dann langsam auf ca. 65°C. Nach 1 Stunde kühlt man auf Raumtemperatur ab, trennt das entstandene Chloroform ab und versetzt die klare Lösung mit 40 ml technischer Natriumbisulfitlösung. Durch Zugabe von konz. Salzsäure wird auf pH 2 eingestellt, dann saugt man das ausgefallene Produkt ab und wäscht mit Wasser nach. Man erhält so 190 g (87% der Theorie) 3-Brom-4-fluorbenzoesäure in Form eines farblosen Pulvers mit dem Schmelzpunkt 132°C.

## Patentanspruch

Verfahren zur Herstellung von 3-Brom-4-fluor-benzoesäure der Formel (I)

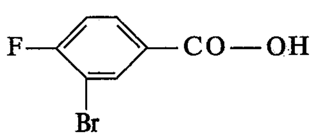 (I)

dadurch gekennzeichnet, daß man Fluorbenzol der Formel (II)

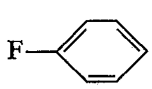 (II)

mit Acetylchlorid in Gegenwart eines Acylierungskatalysators bei Temperaturen zwischen 0 und 100°C umsetzt, das hierbei erhaltene Reaktionsgemisch mit Brom bei Temperaturen zwischen 50 und 150°C umsetzt und das Bromierungsprodukt nach vorheriger Isolierung mit Chlorlauge bei Temperaturen zwischen 0 und 100°C umsetzt.

**Claim**

Process for the preparation of 3-bromo-4-fluoro-benzoic acid of the formula (I)

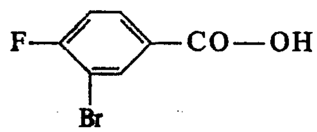

(I)

characterised in that fluorobenzene of the formula (II)

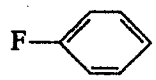

(II)

is reacted with acetyl chloride in the presence of an acylation catalyst at temperatures between 0 and 100°C, the reaction mixture thus obtained is reacted with bromine at temperatures between 50 and 150°C, and the bromination product, after being isolated beforehand, is reacted with hypochlorite solution at temperatures between 0 and 100°C.

**Revendication**

Procédé pour la fabrication d'acide 3-bromo-4-fluoro-benzoïque de formule (I)

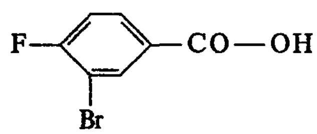

(I)

caractérisé en ce que l'on fait réagir le fluorobenzène de formule (II)

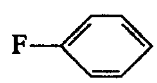

(II)

avec le chlorure d'acétyle à des températures entre 0 et 100°C en présence d'un catalyseur d'acylation, on fait réagir le mélange de réaction ainsi obtenu avec le brome à des températures entre 50 et 150°C et on fait réagir le produit de bromuration, après isolement préalable, avec la lessive de chlore à des températures entre 0 et 100°C.